# EUROPEAN PATENT SPECIFICATION

(11) **EP 1 663 349 B1**
(45) Date of publication and mention of the grant of the patent: **20.02.2008**
(21) Application number: 04785139.9
(22) Date of filing: 22.09.2004
(51) Int. Cl.: A61M 3/02, A61M 35/00, B05B 11/04

(54) **WOUND IRRIGATION DEVICE AND METHOD**
WUNDIRRIGATIONSVORRICHTUNG UND VERFAHREN
DISPOSITIF ET METHODE D'IRRIGATION DE PLAIE

(30) Priority: 22.09.2003 US 504767 P
(43) Date of publication of application: 07.06.2006
(73) Proprietor: Innovation Technologies, Inc., Gainesville, FL 32608 (US)
(72) Inventor: RUCINSKI, Paul, J., Gainesville, FL 32608 (US)
(74) Representative: Perry, Robert Edward
(86) International application number: PCT/US2004/031666
(87) International publication number: WO 2005/030297

(56) References cited:
- US-A- 4 300 555
- US-A1- 2001 037 095

## Description

### Field of the Invention

This invention relates to a wound irrigation device.

### Background of the Invention

In the management and treatment of a wound there are three primary objectives: (1) prevention of infection, (2) preservation and/or restoration of function, and (3) preservation and/or restoration of cosmetic appearance. The most important of these objectives is the prevention of infection. Success in the prevention of infection directly affects the healing process and the degree to which the other two objectives, function and cosmetic appearance, can be preserved and/or restored.

It is known that the number of bacteria, rather than bacterial type, is a critical determinant of whether a wound becomes infected. Experimental evidence suggests that a critical level of bacteria is approximately 10⁵ organisms per gram of tissue. Below this level, wounds heal; at levels greater than 10⁵ bacteria per gram of tissue, wounds often become infected. All traumatic wounds are contaminated by the time the wound is presented to a medical care facility for treatment (Dire, Daniel I. [1990] "A comparison of Wound Irrigation Solutions Used in the Emergency Department," Annals of Emergency Medicine 19(6):704-708). Dirty wounds, or those which have not been treated within six hours, are likely to be contaminated with bacteria at levels that are higher than the critical level. Reducing the number of bacteria in and around the wound is a recognized and accepted means for avoiding infection and expediting wound healing.

Different procedures of wound management have been developed to help decrease the level of bacteria present in a wound, *i.e.*, reduce the incidence of infection. The cleansing of a wound and the site surrounding the wound to remove blood clots, debris, dirt, or other foreign materials that can introduce contaminants, including pathogenic microorganisms, is critical in reducing levels of bacteria in and around the wound. There are numerous wound cleansing procedures presently used by healthcare professionals such as debridement, excision and irrigation. See, for example, Sinkinson, Craig Alan, ed. (1989) "Maximizing A Wound's Potential For Healing," Emergency Medicine Reports 10(11):83-89; Lammers, Richard L. (1991) "Soft Tissue Procedures: Principles of Wound Management," in Clinical Procedures in Emergency Medicine, Roberts and Hedges, eds., 2nd Ed., W.B. Saunders Company, pp. 515-521; Cracroft, Davis (1987) "Minor Lacerations and Abrasions," Emergency Medicine: A Comprehensive Review, Kravis and Warner, eds., 2nd cd., Aspen Publishing Co., pp. 107-110; and Mulliken, John B. (1984) "Management of Wounds," in Emergency Medicine, May ed., John Wiley & Sons, pp. 283-286.

Irrigation is the most commonly used procedure for cleansing of open contaminated wounds. Irrigation involves the application of sterile solutions or fluids to wounds to remove loose devitalized tissue, bacterial inoculum, blood clots, loose debris, and foreign bodies proximate to and within the depths of the wound. The two critical components of any effective wound irrigation method and/or device are: (1) the application of an adequate volume of sterile irrigation solution to the wound, and (2) the use of sufficient pressure applied in an effective dispersal pattern in the delivery of the solution to effectively remove contaminants. Regarding volume, the amount of irrigation solution required will depend upon the type of wound and the level of contamination. Injuries which can introduce a high amount of bacteria into a wound (such as puncture wounds and bites) may require 1 liter or more of irrigation solution. See Mulliken, 1989. Regarding pressure, it has been demonstrated that a minimum stream pressure of 27.6 kPa (4 psi), and preferably 48.3 kPa (7 psi), is required to effectively flush or remove contaminants from a wound. See, for example, Rodeheaver GT. Wound Cleaning, Wound Irrigation, Wound Disinfection, In: Krasner D., Kane D. Chronic Wound Care. 2nd ed. Wayne, P.A.: Health Management Publications; 1997, pp 97-108; and Bergstrom N., Bennett, M.A., Carlson, C.E. *et al.* Treatment of Pressure Ulcers. Clinical Guideline No. 15. AHCPR Publication No. 95-0652. Rockville, MD. Department of Health and Human Services. Public Health Services, Agency of Health Care Policy and Research; December 1994.

Irrigation pressure in excess of desired limits (*e*.*g*. 172.6 kPa (25 psi) or greater) may actually drive bacteria and particulate matter deeper into the wound and thereby defeat the purpose of the irrigation process. High-pressure irrigation may also cause damage to healthy tissue and impede the tissue's defenses and retard healing. Thus, effective wound irrigation requires the use and application of adequate volumes of irrigation solution delivered to the wound in an effective dispersal pattern at appropriate pressures.

Bulb syringes or gravity flow irrigation devices deliver fluid at low pressures and as such are ineffective in ridding wounds of small particulate matter or in sufficiently reducing wound bacterial counts. Irrigation by bulb syringe exerts a pressure of about 0.05 psi, which does not reduce the number of bacteria or particulate contaminants enough to prevent infection. The flow rate of irrigation fluid delivered through intravenous (IV) tubing can be enhanced by inflation of a blood pressure cuff around a collapsible plastic IV bag. This method is cumbersome and provides considerably less irrigation pressure than can be delivered by a plunger-type syringe.

The plunger-type syringe is the most common irrigation device currently used. Its use involves filling the barrel of the syringe with sterile irrigation solution and depressing the plunger to generate and apply a single pressurized stream of solution in and around the wound to dislodge and rinse away contaminants. This device has two notable disadvantages: (1) an extremely limited reservoir of irrigation fluid (typically a syringe with a 35 cc-capacity barrel), and (2) it is limited to dispersal and application of a single concentrated stream of solution to the wound. Consequently, in most cases, the syringe must be repeatedly refilled in order to apply sufficient quantities of irrigation solution to a wound. This is time-consuming and cumbersome to do while attempting to maintain a sterile field. In an attempt to address this limitation, a device has been developed that involves a system consisting of a syringe and IV tubing with a valve system that attaches to a bottle of saline to provide a ready means of refilling the syringe barrel. (Travenol pressure irrigation set, code no. 2D2113, or irriget, Ackrad Laboratories, Garwood, NJ). Additionally, U.S. Patent No. 4,357,937 describes a disposable, manually operable medical irrigation device which is adapted for providing selective volume and stream intensity in liquid flow from a plurality of syringes. These devices do not adequately address the disadvantages of using syringes for irrigation as discussed above and are not commonly used in clinical practice due to their complexity of use and cost.

The amount of hydraulic pressure that can be delivered with a plunger-type syringe varies with the force exerted on the plunger of the syringe and with the internal diameter of the attached needle. Plunger-type syringe devices that deliver moderate pressure employ either a 19 gauge needle attached to a 35 cc syringe, which creates hydraulic pressure in the range of 27.6 - 55.2 kPa (7-8 psi), or a 30 ml syringe fitted with a 19 gauge needle which typically creates about 27.6 kPa (7 psi) irrigation pressure. A 22 gauge needle attached to a 12 cc syringe, delivers a pressure of about 89.6 kPa (13 psi). Such pressures have been proven effective in wound irrigation, but, as stated above, such devices apply only a single concentrated stream of solution to the wound. In addition, these described devices hold less than adequate volumes of irrigation solution and therefore require repeated refilling which is time consuming and cumbersome.

U.S. Patent No. 5,071,104 describes a wound irrigation apparatus and process for cleansing wounds which includes a pressure bladder, e.g., a blood pressure cuff, disposed proximate a reservoir holding a cleaning solution. The device in the '104 patent also includes a flexible tubular conduit for transmitting the solution from the reservoir to a single nozzle. The conduit and reservoir form a two-part system which is time consuming to set up, inconvenient to use, and costly.

U.S. Patent No. 5,133,701 describes a disposable pressurized wound irrigation device which has a pressurized chamber for providing a force upon the reservoir such that a single liquid stream of cleansing solution is expelled from the device at a constant pressure. A propellant is used in evacuating the cleanser contents of the device. This invention requires a propellant and involves a relatively elaborate manufacturing and filling process which is labor intensive and requires specialized machinery. This device is also inconvenient to use and costly.

More recently, an advantageous wound irrigation system has been developed whereby a dispersed stream of irrigation fluid is easily and effectively applied to wounds. This system is described at, for example, U.S. Patents Nos. 5,830,197 and 6,468,253 and International Patent Applications WO 00/15279 and WO 02/007799. Although the use of the dispersed stream was highly advantageous, it has now been determined that the shape and size of the nozzles delivering the irrigation fluid can be improved, to give even better results.

### Summary of the Invention

According to the present invention, a device for wound irrigation comprises a reservoir housing containing a sterile wound irrigation solution, wherein the reservoir housing is attached to a discharge means having at least one nozzle having a passage through which the wound irrigation solution can exit the reservoir housing, the passage has a cross-sectional area that decreases from its upstream end to its downstream end, and the passage includes a portion having a curved surface.

In a preferred embodiment, the device has a plurality of nozzles that are specifically designed to reduce the pressure loss as the irrigation fluid leaves the reservoir housing. There are two elements of the design that are particularly important, i.e. the shape of the nozzle and the length of the nozzle.

In a preferred embodiment, the reservoir housing, upon which a discharge means is either permanently or detachably affixed, is compressible (e.g., plastic bottles in which saline solutions are presently available). The operator (i.e. medical or health care professional or other person) using the device and providing wound irrigation therapy can easily compress the reservoir housing to force the irrigation solution through the nozzle(s) of the discharge means under sufficient pressure to dislodge dirt, debris or other particles, including microorganisms, e.g. pathogenic bacteria.

Specifically exemplified herein is the use of a plurality of elongated nozzles to achieve the desired dispersal, pressure and volume of the stream of irrigation solution.

The present invention provides an easy to use, economical wound irrigation system that is capable of delivering adequate volumes of irrigation solution (without refilling the reservoir) in a dispersed stream under sufficient pressure to effectively cleanse the wound, thereby reducing the incidence of infection.

### Brief Summary of the Figures

**Figure 1** shows the subject wound irrigation device that includes a compressible reservoir housing, and a discharge means that has a plurality of nozzles for directing a pressurized stream of irrigation solution to a wound.
**Figure 2** shows an embodiment of the subject wound irrigation device with a protective cover.
**Figure 3** shows one embodiment of the subject wound irrigation device.
**Figure 4** shows elongated nozzles of the subject invention.
**Figure 5** shows a specific embodiment of the subject invention.
**Figure 6** shows a venturi shaped nozzle of the subject invention.

### Detailed Description of the Invention

The subject invention provides novel, convenient, inexpensive, and effective wound irrigation devices that comprise, in a preferred embodiment, a reservoir housing and a discharge means having one or more nozzles for irrigation of a wound.

The use of a device of the present invention makes it possible to conveniently and easily apply a stream of irrigation fluid to a wound with the stream having an appropriate volume, pressure and dispersal pattern. Under optimal circumstances, a device of the present invention is utilised by trained emergency medical technicians; however, because of the simplicity and convenience of the device, it can be used to greatly enhance the effectiveness of wound irrigation regardless of the training level of the operator performing the irrigation.

In a preferred embodiment, each nozzle acts as a jet through which irrigation fluid is forced, under pressure, to achieve velocities and pressures appropriate for efficient wound (including eye) irrigation. The nozzles are designed to reduce friction and turbulence and facilitate achieving sufficient irrigation pressures with minimal operator effort.

In accordance with the subject invention, it has been determined that an elongated nozzle is preferred. As used herein, reference to the "nozzle" refers to a passage 98 that delivers a stream of irrigation fluid from the inside to the outside of a reservoir housing. In accordance with the subject invention, the nozzle is preferably longer than 0.127 mm (0.005 inches) from inlet port 102 to outlet port 96. More preferably, the nozzle is longer than 0.254 mm (0:01 inches). Also, it has been determined that it is preferable for the nozzle to be less than 12.7 mm (0.05 inches). More preferably, the nozzle is less than 7.62 mm (0.3 inches). Even more preferably, the nozzle is about 5.08 mm (0.2 inches) long.

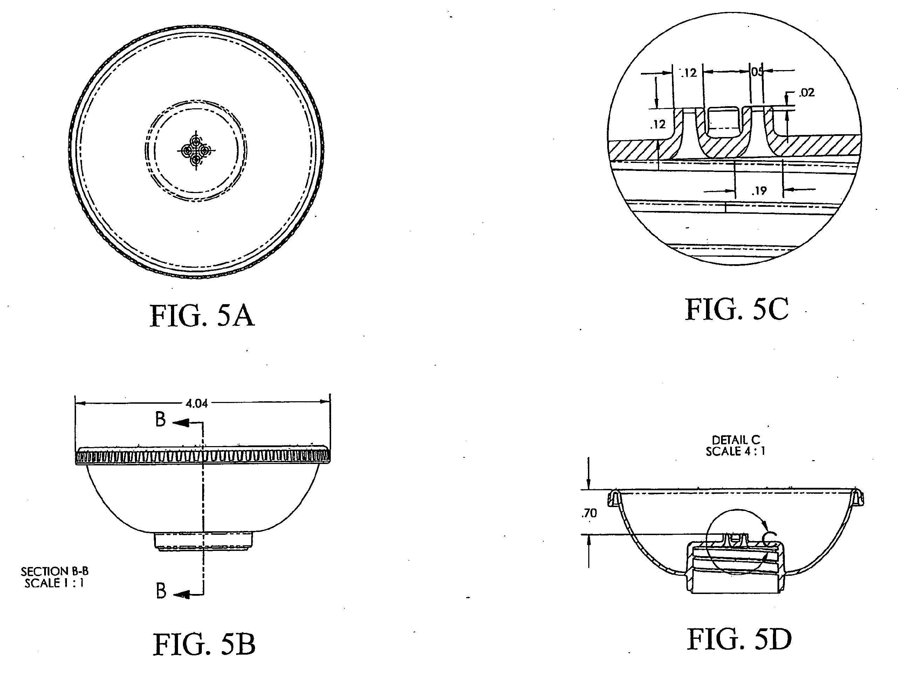

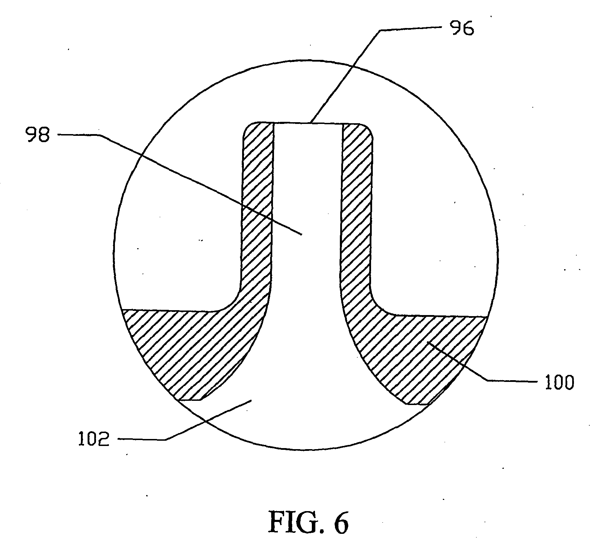

In certain embodiments of the invention (see Figures 5 and 6), the nozzle is defined by a cylindrical bore 98 that narrows as it approaches the outlet port 96. The shaped passageway of the nozzle limits the generation of turbulence in the irrigation fluid as it passes through the nozzle during the operation of the wound irrigation device. Therefore fluid passing through the nozzle experiences laminar flow (or at least a reduction in turbulence) as it passes through and exits the nozzle. The turbulence through the nozzle is less than the turbulence of a nozzle of the same or similar size having a "squared-off" inlet port and/or constant diameter passageway. This shaped nozzle has been found to be particularly advantageous for achieving desired irrigation fluid pressures and velocities.

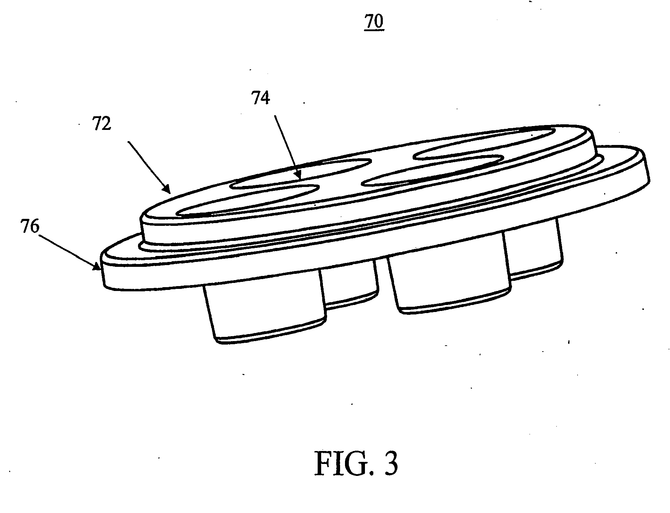

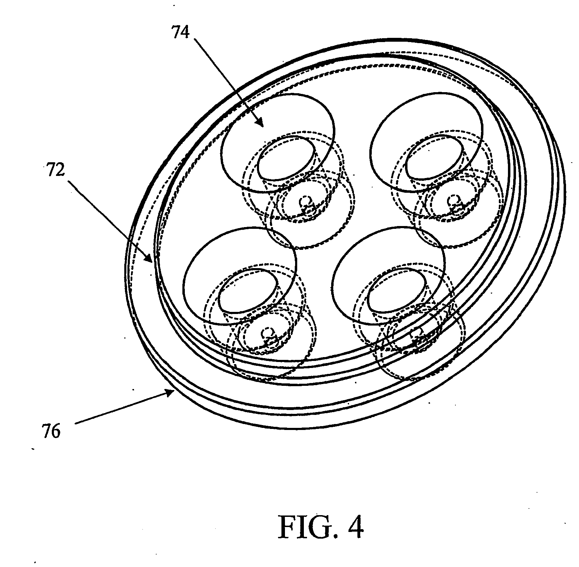

As would be appreciated by a person skilled in the art having the benefit of the current disclosure, the nozzles in a device of the present invention can be formed within the material of the discharge means. Thus, if the discharge means is formed of plastics that is sufficiently thick, then the nozzles may simply pass through the material of the discharge means. Alternatively, the nozzles may extend from either side of the discharge means. Such an embodiment is shown in Figures 3-5.

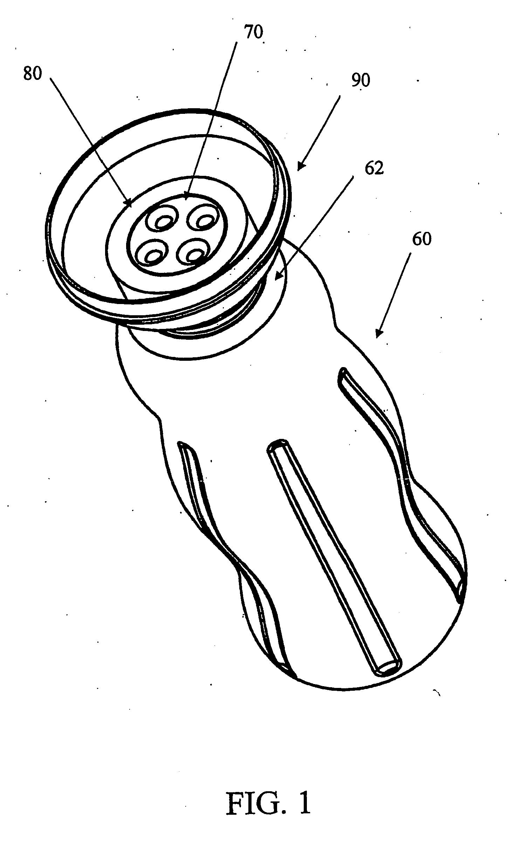

Figure 1 shows an embodiment of the subject invention wherein the device comprises a squeezable reservoir housing having a wall 60 that forms a reservoir that can contain therein an irrigation material (such as wound-cleaning material). The reservoir can preferably hold a liquid solution (for example, sterile saline) as the wound cleansing solution for irrigating, and thereby removing, particles or other contaminants from a wound. The reservoir housing has a mouth 62, which communicates the reservoir to the outside of the housing. Disposed over the reservoir housing mouth, and affixed to the reservoir housing mouth is a discharge means 80, 100.

Another embodiment of the subject invention includes a reservoir housing comprising an inlet port and fitting for attaching tubing for delivery of pressurized gas to the reservoir. Pressure sources generally available in hospitals, emergency rooms, and other medical clinics or facilities provide a pressure of up to 379 kPa (55 psi). The reservoir can be attached by, for example, a flexible tube to the pressure source connector and to a fitting provided on the reservoir housing of the subject device.

In certain embodiments, the discharge means is detachably affixed to the reservoir housing mouth. In such embodiments, the reservoir mouth can include connecting means such as threads, snap fits, grooves, or other mechanical connection configurations for operably connecting the reservoir housing mouth to the discharge means.

The wall of the reservoir housing can be made or molded from any material that is preferably rigid enough to stand upright when the reservoir is filled with irrigation solution. In a typical embodiment, the reservoir housing is formed by a molded plastic, which is pliable enough so that the wall of the reservoir housing can be squeezed or compressed by hand to exert pressure on the contents of the reservoir. The preferred embodiment comprises a plastic material that is pliable enough to squeeze by hand and which also has sufficient resilience to return to its original shape when no longer compressed or squeezed.

The horizontal cross-sectional shape of the reservoir housing can be circular, square, rectangular, or other geometric shapes as desired or as already available. The walls can be tapering toward one end or the other. Alternatively, other shapes can be made for the reservoir housing according to and adapted for a particular use. For example, part of the reservoir housing wall can be slightly rounded as in a general hourglass shape and/or can be molded for ergonomics to easily fit a hand or otherwise to facilitate handling or compressing the reservoir housing. The reservoir formed by the housing of the subject invention can typically hold a volume of about 100 ml to 1000 ml, preferably about 250 ml to about 750 ml and most preferably about 500 ml. Advantageously, with manual compression, the device and method of the subject invention can deliver 500 ml of irrigation fluid in less than 30 seconds and, typically, in 15 to 25 seconds. The fluid is delivered at 27.6 to 137.9 kPa (4 to 20 psi). Lower pressures can be used for irrigating eye wounds. For irrigation of wounds in or around the eye, a pressure of 6.9 to 34.5 kPa (1 to 5 psi) is preferred.

Further, in a preferred embodiment, the reservoir housing comprises at one end a neck portion formed at the mouth of the reservoir housing. The neck portion of the reservoir housing is generally at least slightly smaller in cross sectional area than the reservoir housing. The reservoir housing neck is preferably integrally molded with the reservoir housing, but can be formed or molded separately and affixed to the mouth of the reservoir housing. The material used for the neck portion of the reservoir housing can be the same as the material used to make the reservoir housing cylinder. Alternatively, the neck portion can be a different material, for example, a more rigid or sturdy material than the compressible material forming the reservoir housing wall. For example, the material used to make the neck portion can be a metal or a hard plastic, or the like.

With reservoir housing embodiments that include a neck portion, the discharge means is typically disposed over and affixed to the neck portion. In a related embodiment, the neck portion of the reservoir housing can include a connecting means for detachably affixing a discharge means thereto. The connecting means can include threads, latches, grooves, or other mechanical connection configurations for operably connecting the neck portion to the discharge means. The connecting means can be on the outer face of the neck portion, forming a male connecting end, or can be on the inner face forming a female connecting end of the neck portion.

In a preferred embodiment, the discharge means has a plurality of elongated nozzles 70 whereby the irrigation solution in the reservoir passes through in a pressurized and directional manner. A backsplash shield 90 can also be provided either with the reservoir housing or with the discharge means.

The back-splash protective shield protects the health care professional (or other user) from back-splash of human and or animal body fluids that are mixed with and splashed from the wound when the wound is contacted by the discharged irrigation solution.

As described herein, a critical feature of the subject invention is the unique design of the nozzles that enable easy and convenient creation of a dispersed stream of irrigation solution having the appropriate volume, pressure and dispersal pattern to obtain effective wound irrigation.

As used herein, reference to a "dispersed" stream of solution means that the area from which the stream emanates, or the area which it contacts, is larger than that which can be achieved using a typical syringe for irrigation. A typical syringe, as is well known in the art can be, for example, a 16 or 18 gauge syringe. In one embodiment, the dispersed stream can be achieved using multiple nozzles. The nozzles can be presented in a variety of patterns on a discharge means, such as a circular or square pattern.

The discharge means with nozzles is a particularly advantageous feature of the invention. The discharge means can be, for example, a flat or domed disc of approximately the same size as the opening of the reservoir housing. In one embodiment, the discharge means has a plurality of nozzles.

In certain embodiments, the discharge means is designed with connecting means that are threads or grooves, which allow for complementary attachment to currently available irrigation solution bottles. Thus, the discharge means of the subject invention can be interchangeable, when desired, with the screw-cap that is provided with an irrigation solution bottle as are available. The screw-top design of the discharge means provides the operator with the option of using the reservoir housing with the nozzles of the invention or to threadably remove the discharge means and pour out or change the irrigation solution.

Each of the nozzles of the discharge means can be of any desirable size, preferably less than one-eighth inch in diameter and having a size between about a 10 gauge hypodermic needle and about a 30 gauge needle, and most preferably having a size ranging from that of a 16 gauge needle to a 25 gauge needle. Specific dimensions and shapes are shown in Figure 5. The outlet port 96 may have, for example, an inner diameter of 0.508 to 0.178 mm (0.02 to 0.07 inches). For the venturi shaped nozzle (Figure 6), the diameter of the inlet port **102** (proximal to the reservoir) can be, for example, from 1.27 to 7.62 mm (0.05 to 0.30 inches), or more.

Each of the nozzles can be the same size or the nozzles can be different sizes and shapes. The different sizes of nozzles allow for the liquid to be expelled from the discharge means at different pressures. For example, the 16 gauge nozzle allows for a stream having about 41.4 kPa (6 psi) pressure when the device is squeezed by the normal adult; the 25 gauge nozzle provides a pressure of up to about 137.9 kPa (20 psi) from each nozzle.

The shaped nozzles of the invention have the added advantage when compared to other nozzles in that little or no release of irrigation material is permitted without pressure being applied to the irrigation material. For example, if a reservoir housing with shaped nozzles is tipped onto its side or even held upside-down with gravitational pull on the irrigation material through the discharge means, there will be little or no release of irrigation material through the shaped nozzles.

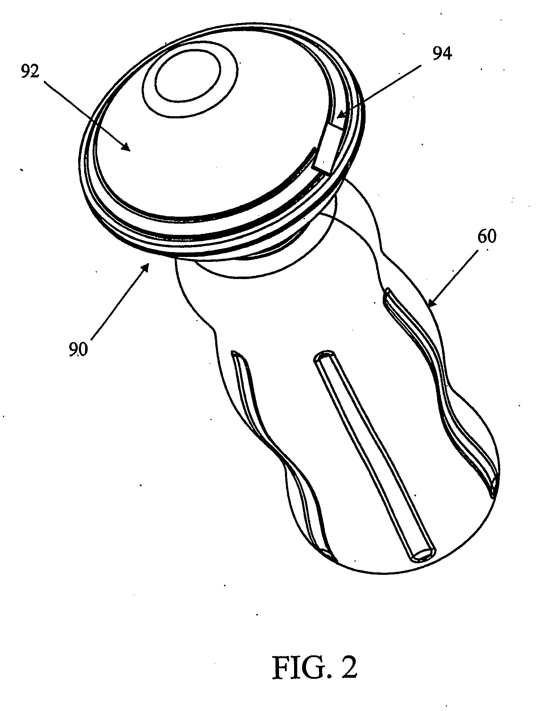

As shown in Figure 2, one embodiment of the subject invention also includes a removable or partially detachable protective cap 92, which is placed over the discharge means to protect the nozzles and contents of the reservoir from contamination or premature discharge or leakage. The protective cap can be attached with an element 94 that is removable. Such elements 94 include fasteners, non-permanent adhesives, and the like.

In a preferred embodiment, the discharge means 70 comprises four nozzles. Additionally, to discharge the irrigation solution at appropriate pressure, the diameter of the nozzles 74 can be 0.508 to 1.778 mm (0.02 to 0.07 inches) in diameter.

The irrigation solution used can be water, saline, or a balanced salt solution. The solution is preferably sterile and at the discretion of the user or manufacturer of the irrigation solution can additionally comprise an antibacterial and/or antifungal component. The device can be sterilized by known sterilization techniques, including boiling, autoclaving, gas sterilization and the like, either separately or together with the reservoir housing.

Buffered Ringer's solution or commercially available balanced salt solution (e.g., Tis-U-Sol or Physio-Sol) are physiologically compatible and are commonly used in wound irrigation procedures.

The antiseptic agents most commonly used in wound care at present include:
Povidone-iodine solution (Betadine preparation)-iodine added to the carrier polyvinylpyrrolidone (PVP), a water-soluble organic complex; this combination is called an iodophor. Standard solutions of Betadine preparation are 10 per cent.
Povidone-iodine surgical scrub (Betadine scrub)-the iodophor PVP-I and an anionic detergent (pH 4.5).
pHisoHex-an emulsion of an anionic detergent, entsulfon, lanolin cholesterols, petrolatum, and hexachiorophene (pH 5.5).
Hi-Bi-clens-chlorhexidine gluconate plus a sudsing base (pH 5.1 to 6.5).
Tincture of green soap-potassium oleate, isopropanol, potassium coconut oil, soap.
Dakin's solution 0.2 per cent solution hypochlorite solution.
Hydrogen peroxide-an oxidizing agent.
Benzalkonium chloride (Zephiran)-a quaternary ammonium compound that works as a cationic surface active agent.
Nonionic surfactants-Pluronic F-68 (Shur-Clens) and Poloxamer-188 (Pharma Clens)-agents that have no antimicrobial activity (pH 7.1).

From the description of the device herein above, a method of using the subject device would readily be understood and adaptable by those persons having ordinary skill in the art. The reservoir housing is filled with a desired irrigation solution. The irrigation solution is sterilized before or after filling. The reservoir housing and contents can be stored in a sterile environment, e.g., sterile packaging which is opened immediately prior to use. In a preferred use, the protective shield is removed, then the reservoir housing can be directed towards the wound and squeezed or compressed to expel or discharge the solution in the desired direction, and at the desired pressure to effect irrigation of a wound to remove contaminants or debris. See also the Example 2, provided below.

It would also be understood that the described discharge means can be packaged separately from the reservoir housing. The discharge means is packaged in a sterile environment. In a preferred use of the embodiment wherein the discharge means is provided separately from the reservoir housing, the cap of a readily available, squeezable irrigation bottle containing a sterile irrigation solution, e.g., normal saline, is replaced with the subject discharge means. The bottle, now having the subject discharge means attached or engaged thereto, can be used as described herein.

In one embodiment, the discharge means is provided in a sterile laceration tray. According to the subject invention, the laceration tray has, in addition to a discharge means or entire irrigation bottle of the subject invention, other items conveniently provided for treating wounds. Contemplated items that can be included in a laceration tray include, but are not limited to, needle holders (*i*.*e*., 127 mm or 5" floor-grade smooth); scissors (*i*.*e*., 104 mm or 4.5" floor-grade straight Iris scissors); hemostats (*i*.*e*., 127 mm or 5" floor-grade curved mosquito hemostat); forceps (*i*.*e*., floor-grade tissue forceps with 1x2 teeth); cups (*i*.*e*., 2 oz. medicine cups); syringes (*i.e*., 10cc Luer Lock syringe); needles (*i*.*e*., 25 gauge x 15.9 mm or 5/8" needle; 27 gauge x 38 mm or 1.5" needle; 18 gauge x 38 mm or 1.5" needle); dressings (*i*.*e*., gauze dressings); drapes (*i.e.,* polylined fenestrated drapes); and towels (*i.e.,* absorbent towels).

Another embodiment of the invention provides a pressurized irrigation assembly to provide automated dispersal of irrigation solution. The pressurized irrigation assembly can comprise: irrigation solution; a reservoir housing that contains the irrigation solution; a discharge means having a plurality of specifically designed nozzles through which a sufficient volume of the irrigation solution can pass at an appropriate pressure; a means for creating irrigation solution pressure for the generation of a plurality of dispersed streams through the nozzles to irrigate damaged tissue.

A variety of pressure means have been developed to enable automatic (as opposed to manual) transfer of irrigation solution from a reservoir housing to damaged tissue. For example, U.S. Patent No. 6,574,527 to Henniges *et al.* describes a hand held irrigator that can be attached to the mouth of a reservoir housing irrigation solution. Various other apparatus that enable the automatic transfer of irrigation solution from a reservoir housing to damaged tissue include, but are not limited to, U.S. Patents Nos. 6,751,813; 6,746,419; 6,106,494; 5,484,402; 5,470,305; 5,269,750; and 5,046,4861.

In one embodiment of the invention, the pressure means is a hand-held device similar to the irrigator disclosed in U.S. Patent No. 6,754,527. The hand held device has a tip and a supply end. Irrigation solution from the reservoir housing is provided to the supply end of the pressure means and is eventually discharged from the tip of the pressure means. Affixed to the tip is a discharge means of the invention, which can be detachably affixed to the tip. The hand held device further comprises a pump for regulating the rate of irrigation solution discharge and a motor for actuating the pump. In certain embodiments, the motor is a battery operated motor.

In a method of use, where a reservoir housing **60** having discharge means **70** affixed thereto is provided, a protective cap **92** is first removed from the backsplash shield **90.** The discharge means **70** is directed towards the wound, and the reservoir housing **60** is compressed, discharging the irrigation solution through the discharge means **70.** The solution can be discharged at a range of pressures of 55.2-157.9 kPa (4-20 psi), with a preferred pressure of about 48.9 kPa (7 psi).

The reservoir housing **60** can be compressed manually or via other mechanical means. For example, the operator may compress the reservoir housing using either one hand or two hands, to provide increased pressure (*i.e.,* 110.3 kPa or 16 psi). Alternatively, a pressure means can be activated to generate a dispersed stream of irrigation solution through the discharge means.

In another method of use, where a reservoir housing **60** and discharge means **70** are provided separately, a protective cap **92** is removed from the mouth or neck portion of the reservoir housing. The discharge means is then affixed to the mouth or neck portion of the reservoir housing via complementary connecting means. After the discharge means is affixed to the reservoir housing, the discharge means is directed towards the wound or eye, and the reservoir housing is compressed to discharge a dispersed stream of irrigation solution through the nozzles of the discharge means.

Significantly, it is known that more force is required to rid the wound of particles with a small surface area (*e.g*., bacteria) than to remove particles with a large surface area (*e.g*., dirt, sand, or vegetation). Minimum recommended volumes of irrigation solution vary, but for a moderately sized potentially contaminated wound, for example a laceration 3-6 cm long and less than 2 cm deep, at least 200 to 500 ml, or more should be used. Greater volumes, on the order of one to two liters, may be required for larger or heavily contaminated wounds. Irrigation should continue at least until all visible, loose particulate matter has been removed.

Following are examples that illustrate procedures for practicing the invention. These examples should not be construed as limiting.

### Example 1 - Preferred Nozzle Designs

Performance of nozzle designs with regard to pressure, flow rate and dynamic pressure, were compared.

In one test, a squeeze bottle with inlet fitting and pressurized reservoir was set up on its side and filled. A flow meter was placed between the squeeze bottle and the reservoir. A test pressure range of 13.8 - 48.3 kPa (2-7 psi) was applied to each nozzle design and the flow rate was recorded for each test pressure (increments of 6.9 kPa or 1 psi).

The shaped nozzle design (defined by a venturi shaped passageway) created greater flow than the sharp edged, non-shaped nozzle at the same fluid pressure.

For a pressure test, a fulcrum and scale were set-up in front of the horizontally mounted irrigation device. The pressure to the nozzles was varied from 13.8 to 48.3 kPa (2 to 7 psi) and the force was recorded at each 6.9 kPa (1 psi) increment.

The shaped nozzle produces between 70% - 200% more force than the sharpedged nozzle.

At 27.6 kPa (4 psi) the calculations show 25.7 and 19.2 grams of force for the shaped and non-shaped nozzles, respectively.

Thus, the discharge means with 4-venturi shaped nozzles produces more force and more flow per squeeze on the irrigation bottle than the discharge means with 4 non-shaped nozzles.

### Example 2 - Methods of Wound Irrigation

When a patient presents a wound to a medical or other health care professional skilled in the art, that medical professional assesses the extent of the injury sustained by the patient, including all other life threatening injuries. Appropriate action regarding these life threatening injuries is performed and a history is recorded. All wounds are covered to minimize further contamination until the actual repair process begins.

For examination of the wound, it is assumed that a medical professional would have performed a detailed evaluation of the extent of tissue injury, including but not limited to: anatomical area considerations, depth of the wound, type of injury, *e.g.,* crash injury, puncture wound, bites, missiles, cuts with sharp objects, or the like. Included in this examination would be a determination of the type(s) of contamination, time elapsed between the occurrence of the injury to presentation, gross contamination of a wound, and other medical factors associated with an increase incidence of infection (for example, diabetics, AIDS patients, and chemotherapeutics patients).

The wound and surrounding tissue, at the option of the health care professional, could be anesthetized using topical, local, or general anesthetics before the wound-cleansing method begins.

In one embodiment, the subject device has a discharge means affixed to a reservoir housing as described with a protective shield covering the discharge means. The health care professional using the subj ect device would remove the protective shield to expose the discharge means. The subject device can be held in either hand as preferred by the user. Normally, it would be held in the dominant hand in a bottle-holding fashion. This allows the medical care professional to gently open the wound if needed, with the opposite hand, preferably protected by a sterile glove, to expose the depths of the wound.

Once the depths of the wound have been exposed, the end of the reservoir housing having the discharge means affixed thereto is directed towards the wound. Manual or mechanically produced pressure is applied to the reservoir housing to expel the irrigation solution through the nozzles of the discharge means. The wound should be irrigated in this fashion until all visible evidence of contamination has been removed. A potentially contaminated wound of any size should be irrigated with a minimum of 200-300 ml of irrigation solution. Heavily contaminated or larger wounds may require 2-3 liters of irrigation solution. The health care professional could vary the angle of the discharged irrigation solution from the discharge means in reference to the wound to further assist with the dislodgement of contaminants. This variation in the angle will also decrease or increase the amount of back-splash. Thus it would be important to irrigate in a manner that decreases the back-splash. Minimizing back-splash is achieved by irrigation at acute angles to the plane of the wound.

Following an initial irrigation of the wound, a re-examination of the wound should be undertaken. The wound should be explored to its base to ascertain that no visible foreign bodies or contaminants remain. If foreign bodies or contaminants are found, the irrigation process should be repeated followed by a re-examination. This may continue for several cycles.

Once irrigation has been completed, *i.e*., no visible contaminants remain, the damaged tissue would be repaired in a standard accepted fashion.

Irrigation of skin wounds such as cuts, scrapes, punctures, abrasions, etc. are particular well-suited for irrigation using a device according to the subject invention.

## Claims

1. A device for wound irrigation, which comprises a reservoir housing containing a sterile wound irrigation solution, wherein the reservoir housing is attached to a discharge means having at least one nozzle having a passage (98) through which the wound irrigation solution can exit the reservoir housing, **characterised in that** the passage has a cross-secti nal a rea that decreases from its upstream end (102) to its downstream end (96), and the passage includes a portion having a curved surface.

2. A device, according to claim 1, wherein the passage is between 0.127 and 12.7 mm (0.005 and 0.50 inches) long.

3. A device according to claim 2, wherein the passage is between 0.254 and 7.62 mm (0.01 and 0.3 inches) long.

4. A device according to claim 1, wherein the passage has an inner diameter of between 0.508 and 1.778 mm (0.02 and 0.07 inches) at the downstream end and between 1.27 and 7.62 mm (0.05 and 0.30 inches) at the upstream end.

5. A device according to claim 1, further comprising a backsplash shield.

6. A device according to claim 1, wherein the reservoir housing is made from a compressible material.

7. A device according to claim 6, wherein the reservoir housing is made from plastic.

8. A device according to claim 1, wherein the reservoir housing is generally in the shape of a cylinder having an ergonomic shape with curved sides such that the cross-sectional area of the cylinder varies along the length of the cylinder.

9. A device according to claim 1, further comprising means for pressurising the reservoir housing.

10. A device according to claim 9, wherein the means for pressurising the reservoir housing is battery-operated.

11. A device according to claim 1, having a plurality of the nozzles.

12. A device according to claim 11, having 4 nozzles.

13. A device according to claim 1, which creates a dispersed stream of irrigation liquid when the irrigation fluid leaves the device under pressure.

14. A device according to claim 1, wherein the reservoir contains approximately 500 ml of the irrigation solution.

15. A device according to claim 1, which is sterile and enclosed within a wrapper that maintains sterility until the wrapper is opened.

## Patentansprüche

1. Vorrichtung zur Wundspülung, welche ein Behältergehäuse umfasst, das eine sterile Wundspülungslösung enthält, wobei das Behältergehäuse an ein Ausstoßmittel mit mindestens einer Düse mit einem Durchlass (98) angeschlossen ist, durch welchen die Wundspülungslösung das Behältergehäuse verlassen kann, **dadurch gekennzeichnet, dass** der Durchlass eine Querschnittsfläche hat, die von seinem vorgelagerten Ende (102) zu seinem nachgelagerten Ende (96) abnimmt und der Durchlass einen Abschnitt mit einer gekrümmten Oberfläche einschließt.

2. Vorrichtung nach Anspruch 1, wobei der Durchlass zwischen 0,127 und 12,7 mm (0,005 und 0,50 Zoll) lang ist.

3. Vorrichtung nach Anspruch 2, wobei der Durchlass zwischen 0,254 und 7,62 mm (0,01 und 0,3 Zoll) lang ist.

4. Vorrichtung nach Anspruch 1, wobei der Durchlass einen inneren Durchmesser zwischen 0,508 und 1,778 mm (0,02 und 0,07 Zoll) am nachgelagerten Ende und zwischen 1,27 und 7,62 mm (0,05 und 0,30 Zoll) am vorgelagerten Ende hat.

5. Vorrichtung nach Anspruch 1, des weiteren einen Rückspritzschirm umfassend.

6. Vorrichtung nach Anspruch 1, wobei das Behältergehäuse aus einem kompressiblen Material hergestellt ist.

7. Vorrichtung nach Anspruch 6, wobei das Behältergehäuse aus Kunststoff hergestellt ist.

8. Vorrichtung nach Anspruch 1, wobei das Behältergehäuse im Allgemeinen in der Form eines Zylinders ist mit einer ergonomischen Form mit gekrümmten Seiten, so dass die Querschnittsfläche des Zylinders über die Länge des Zylinders veränderlich ist.

9. Vorrichtung nach Anspruch 1, des weiteren Mittel zum unter Druck Setzen des Behältergehäuses umfassend.

10. Vorrichtung nach Anspruch 9, wobei das Mittel zum unter Druck Setzen des Behältergehäuses batteriebetrieben ist.

11. Vorrichtung nach Anspruch 1, mit einer Mehrzahl der Düsen.

12. Vorrichtung nach Anspruch 11, mit vier Düsen.

13. Vorrichtung nach Anspruch 1, welche einen zerstäubten Strom Spülflüssigkeit erzeugt, wenn das Spülfluid die Vorrichtung unter Druck verlässt.

14. Vorrichtung nach Anspruch 1, wobei der Behälter ungefähr 500 ml der Spüllösung enthält.

15. Vorrichtung nach Anspruch 1, welche steril und eingeschlossen in einer Verpackung ist, die Sterilität erhält bis die Verpackung geöffnet wird.

## Revendications

1. Dispositif pour l'irrigation d'une plaie, qui comprend un logement de réservoir contenant une solution stérile d'irrigation de plaie, où le logement de réservoir est attaché à un dispositif de décharge ayant au moins une buse présentant un passage (98) par lequel la solution d'irrigation de plaie peut sortir du logement de réservoir, **caractérisé en ce que** le passage a une surface de coupe transversale qui diminue depuis son extrémité en amont (102) vers son extrémité en aval (96) et le passage comprend une partie ayant une surface incurvée.

2. Dispositif selon la revendication 1, dans lequel le passage est long de 0,127 à 12,7 mm (0,005 à 0,50 pouce).

3. Dispositif selon la revendication 2, dans lequel le passage est long de 0,254 à 7,62 mm (0,01 à 0,3 pouce).

4. Dispositif selon la revendication 1, dans lequel le passage a un diamètre interne allant de 0,508 à 1,778 mm (0,02 à 0,07 pouce) à l'extrémité en aval et allant de 1,27 à 7,628 mm (0,05 à 0,30 pouce) à l'extrémité en amont.

5. Dispositif selon la revendication 1, comprenant en outre, un écran pour les éclaboussures.

6. Dispositif selon la revendication 1, dans lequel le logement de réservoir est constitué d'un matériau compressible.

7. Dispositif selon la revendication 6, dans lequel le logement de réservoir est constitué d'une matière plastique.

8. Dispositif selon la revendication 1, dans lequel le logement de réservoir à une forme générale de cylindre ergonomique, avec des côtés courbes de sorte que la coupe transversale du cylindre varie sur la longueur du cylindre.

9. Dispositif selon la revendication 1, comprenant en outre, des moyens pour la pressurisation du logement de réservoir.

10. Dispositif selon la revendication 9, dans lequel le moyen pour la pressurisation du logement de réservoir fonctionne sous piles.

11. Dispositif selon la revendication 1, ayant une série de buses.

12. Dispositif selon la revendication 11, ayant 4 buses.

13. Dispositif selon la revendication 1, qui crée un courant dispersé du liquide d'irrigation, lorsque le fluide d'irrigation quitte la dispositif sous pression.

14. Dispositif selon la revendication 1, dans lequel le réservoir contient environ 500 ml de la solution d'irrigation.

15. Dispositif selon la revendication 1, qui est stérile et disposé dans une enveloppe qui conserve la stérilité jusqu'à ce que l'enveloppe soit ouverte.
